# EUROPÄISCHE PATENTSCHRIFT

(11) **EP 2 934 754 B1**
(45) Veröffentlichungstag und Bekanntmachung des Hinweises auf die Patenterteilung: **09.08.2017**
(21) Anmeldenummer: 13780108.0
(22) Anmeldetag: 16.10.2013
(51) Int. Cl.: B01L 9/00, B81B 7/00, G01F 15/14, B01L 3/00, G01D 11/30, G01N 33/00

(54) **MESSANORDNUNG MIT EINEM TRÄGERELEMENT UND EINEM SENSOR**
MEASURING SYSTEM HAVING A CARRIER ELEMENT AND A SENSOR
SYSTÈME DE MESURE COMPRENANT UN ÉLÉMENT SUPPORT ET UN CAPTEUR

(30) Priorität: 21.12.2012 DE 102012112975
(43) Veröffentlichungstag der Anmeldung: 28.10.2015
(73) Patentinhaber: Endress+Hauser Flowtec AG, 4153 Reinach (BL) (CH)
(72) Erfinder: WIEST, Achim, 79576 Weil am Rhein (DE); HUBER, Christof, CH-3007 Bern (CH); FETH, Hagen, 79106 Freiburg (DE); STEINHOFF, Frank, 79219 Staufen im Breisgau (DE)
(74) Vertreter: Andres, Angelika Maria
(86) Internationale Anmeldenummer: PCT/EP2013/071617
(87) Internationale Veröffentlichungsnummer: WO 2014/095115

(56) Entgegenhaltungen:
- WO-A1-2010/102403
- DE-A1-102010 020 264
- US-A1- 2002 190 839
- US-A1- 2005 042 149
- US-A1- 2009 145 198
- US-A1- 2009 173 166
- US-B1- 7 569 127

## Beschreibung

Die vorliegende Erfindung betrifft eine Messanordnung umfassend ein Trägerelement und einen Sensor nach dem Oberbegriff des Anspruchs 1. US 2005/042149 A1 offenbart eine fluidische Anordnung zur chemischen Synthese, umfassend ein Trägerelement mit Fluidkanal und weitere modulare Element(e), wobei es sich dabei um einen Sensor handeln kann. Hierbei dienen O-Ring-Dichtungen als Anschlusselemente, die auf dem Trägerelement aufliegen und damit senkrecht hervorstehen. Da sie in die Öffnungen hineingelegt werden, ragen sie außerdem in den Fluidkanal des Sensors hinein.
Eine gattungsgemäße Messanordnung eines Trägerelements mit einem mikromechanischen Sensor wird in der DE 10 2011 119 472 B3 offenbart. Diese Messanordnung weist insbesondere in Fig.1 eine Verbindung zwischen dem Trägerelement und dem mikromechanischen Sensor auf, welche durch Lötungen geschaffen wird. Die DE 10 2011 119 472 B3 offenbart zudem, dass die als Chiphalterung bezeichneten Lötungen zudem mit einer zu- und/oder abführenden Leitung versehen sind. Die Lötungen in Fig. 1 und 2 dieser Druckschrift sind allerdings idealisiert dargestellt. Vielmehr ist die Verteilung eines Lotes und dessen Begrenzung ungleichmäßig. Der Bereich der Fluidüberleitung zwischen mikromechanischem Sensor und Trägerelement besitzt daher nicht bei jedem Modell das gleiche Totvolumen, sondern kann in der Massenfertigung stark variieren. Es ist daher Aufgabe der vorliegenden Erfindung ausgehend von der gattungsgemäßen DE 10 2011 119 472 B3 eine fluidleitende Verbindung zwischen einem Trägerelement und einem mikromechanischen Sensor zu schaffen, mit einem definierterem Totvolumen.

Die vorliegende Erfindung löst diese Aufgabe mit den Merkmalen des Anspruchs 1.

Eine erfindungsgemäße Messanordnung umfasst
a) ein Trägerelement mit einer Längsachse A auf welchem ein Sensor zur Ermittlung einer Prozessgröße eines gasförmigen oder flüssigen Fluids angeordnet ist, und
b) den Sensor
wobei der Sensor einen Fluidkanal aufweiset, welcher sich innerhalb des Sensors erstreckt, und wobei das Trägerelement einen Fluidkanal aufweist, und wobei das Trägerelement zur Verbindung des Fluidkanals des Trägerelements mit dem Fluidkanal des Sensors jeweils zumindest ein Anschlusselement aufweist, welches senkrecht zur Längsachse A aus dem Trägerelement hervorsteht und welches in den Fluidkanal des Sensors hineinragt,
dadurch gekennzeichnet, dass das Anschlusselement einstückig mit dem Trägerelement verbunden ist und der Sensor durch ein Klebsystem oder eine Lotverbindung mit dem Trägerelement verbunden ist. Durch die erfindungsgemäße Messanordnung wird eine fluidleitende Verbindung vom Trägerelement zum Sensor erreicht, welcher gegenüber einer herkömmlichen Lötverbindung ein definierteres Totvolumen aufweist.

Vorteilhafte Ausgestaltungen der Erfindung sind Gegenstand der Unteransprüche. Das Anschlusselement ist einstückig mit dem Trägerelement verbunden. Eine besonders bevorzugtes einstückigausgeformtes Anschlusselement kann mittels eines Urformverfahrens, vorzugsweise mittels Kaltverformung wie z.B. durch ein sogenanntes Drückrollverfahren erreicht werden, wie es aus dem Fahrzeugbau (z.B. der DE 10 2006 011 021 A1) bekannt ist. Alternativ kann auch durch spanende Verarbeitung dieses einstückig-ausgeformte Anschlusselement herausgeformt werden.

Alternativ kann das Anschlusselement vorteilhaft als ein rohrförmiges Bauteil ausgebildet sein, welches in dem Fluidkanal des Trägerelements angeordnet ist. Das Anschlusselement kann in diesem Fall an einer Austrittsöffnung des Fluidkanals eine metallische Anbindungsschicht zur mechanischen Anbindung des Anschlusselements an das Trägerelement aufweist, welche sich über einen Teilbereich einer Oberfläche des Trägerelements erstreckt. Dadurch wird eine verbesserte Druckstabilität der Anbindung des Anschlusselements an das Trägerelement erreicht. Zusätzlich dazu kann sich die besagte metallische Anbindungsschicht über einen Teilbereich einer Oberfläche des Sensors erstrecken, zur mechanischen Anbindung des Anschlusselements an den Sensor. Insgesamt weist somit der Übergang zwischen Sensor, Anschlusselement und Trägerelement eine einheitliche Anbindungsschicht auf.

Diese kann einlagig oder besonders bevorzugt mehrlagig ausgebildet sein.

Im Fall dass das Anschlusselement und das Trägerelement einstückig ausgebildet sind, empfiehlt sich eine metallische Anbindungsschicht lediglich zwischen dem Sensor und dem Anschlusselement.

Die metallische Anbindungsschicht entweder nur zwischen Sensor und Anschlusselement oder zwischen Sensor, Anschlusselement und Trägerelement kann vorteilhaft als eine Lotverbindung ausgebildet sein.

Für eine zuverlässige Lotverbindung und eine gute Reproduzierbarkeit empfiehlt sich das Schmelzen eines Lotdrahtes oder besonders bevorzugt einer vorgestanzten Lotfolie oder einer elektrochemisch abgeschiedenen Lotbeschichtung zur Herstellung der Lotverbindung.

Als chemisch-beständige Materialkomponente der Lotverbindung empfiehlt sich vorteilhaft der Einsatz von Gold und/oder Zinn.

Alternativ oder zusätzlich zur Lotverbindung kann die metallische Anbindungsschicht in Form einer elektrochemisch-abgeschiedenen Metallschicht ausgebildet sein. Elektrochemische Abscheidungsmethoden, insbesondere die galvanische Abscheidung, weisen nur geringe Temperaturbeanspruchung auf, so dass es zu keinen Temperaturspannungen beim Auftragen der Anbindungsschicht kommt.

Es sind verschiedene elektrochemische Abscheidungsmethoden bekannt. Die elektrochemisch-abgeschiedene Metallschicht kann besonders vorteilhaft als galvanische Beschichtung ausgebildet sein. Diese Abscheidungsvariante ist besonders von Vorteil, da durch die Einstellung der Stromdichte im Galvanikbad und die Abscheidungsdauer die Schichtdicke der Anbindungsschicht eingestellt werden kann.

Für ein gezieltes Auftragen der Anbindungsschicht ist es von Vorteil, wenn zwischen dem Trägerelement und der galvanischen Beschichtung eine Schicht aus einem Leitlack angeordnet ist. An dieser Stelle erfolgt eine besonders intensive Abscheidung und damit eine gute Verankerung der Anschlusselemente.

Das Trägerelement und/oder das Anschlusselement sind vorzugsweise aus Metall, vorzugsweise aus Edelstahl, besonders bevorzugt aus Edelstahl der Sorte PH 17-4 besteht. Dieses Material ist einerseits korrosionsfest und andererseits weist es einen an das Sensormaterial angepassten thermischen Ausdehnungskoeffizienten auf.

Insbesondere ist es von Vorteil, wenn die Materialien des Trägerelements derart an das Material des Sensorelements angepasst ist, dass der thermische Ausdehnungskoeffizient des Materials des Trägerelements weniger als das 7-fache, vorzugsweise weniger als das 5-fache des thermischen Ausdehnungskoeffizienten des Materials des Sensors beträgt. Gleiches ist ebenfalls für das Anschlusselement von Vorteil.

Zur zusätzlichen Stabilisierung ist es von Vorteil, wenn zwischen dem Sensor und dem Trägerelement weitere stoffschlüssige Verbindungen angeordnet sind. Diese stoffschlüssigen Verbindungen können insbesondere Lotverbindungen sein.

Besonders von Vorteil ist es, wenn die vorgenannten stoffschlüssigen Verbindungen möglichst gleichmäßig im Bereich zwischen dem Sensor und dem Trägerelement verteilt sind. Daher ist es von Vorteil, wenn die dem Trägerelement zugewandte Oberfläche des Sensors in zumindest drei gleich-dimensionierte Sensorabschnitte einteilbar ist, wobei zumindest zwei der drei Sensorabschnitte zumindest jeweils eine der stoffschlüssigen Verbindungen aufweist.

Die Erfindung wird anhand der nachfolgenden Zeichnungen näher erläutert. Es zeigt:
Fig. 1: eine schematische Darstellung eines Sensors mit Sensorkörper und Anschlussöffnungen,
Fig. 2: eine schematische Darstellung des Sensorkörpers mit einer ersten galvanischen Schicht auf einem Teil der Oberfläche des Sensorkörpers, welche die Anschlussöffnungen elektrisch verbindet,
Fig. 3: eine schematische Darstellung des Sensorkörpers mit Leitungsrohren und Stöpsel, die an den Anschlussöffnungen angeordnet sind,
Fig. 4: eine schematische Darstellung des Sensorkörpers mit Leitungsrohren und Röhren, die an den Anschlussöffnungen angeordnet sind,
Fig. 5: eine schematische Darstellung des Sensorkörpers mit Leitungsrohren, die mittels einer Stütze gegen eine erste Seitenfläche des Sensorkörpers gestützt sind,
Fig. 6: eine schematische Darstellung des Sensorkörpers mit einer galvanischen Beschichtung auf einer Oberfläche des Sensorkörpers und einer Mantelfläche eines jeden Leitungsrohres,
Fig. 7: eine schematische Darstellung des Sensorkörpers mit Leitungsrohren ohne Stöpsel, mit der galvanischen Beschichtung auf der Oberfläche des Sensorkörpers und der Mantelfläche eines jeden Leitungsrohres, und
Fig. 8: eine schematische Darstellung des Sensorkörpers mit Leitungsrohren und Röhren, mit der galvanischen Beschichtung auf der Oberfläche des Sensorkörpers und der Mantelfläche eines jeden der Leitungsrohres.
Fig. 9: eine Schnittansicht eines Trägerelements einer ersten Messanordnung;
Fig. 10: schematische Schnittansicht der Messanordnung;
Fig. 11: schematische Schnittansicht der Messanordnung in der Perspektive;
Fig. 12: schematische Darstellung des Trägerelements der Messanordnung mit einer metallischen Anbindungsschicht; und
Fig. 13: schematische Darstellung einer zweiten Messanordnung.

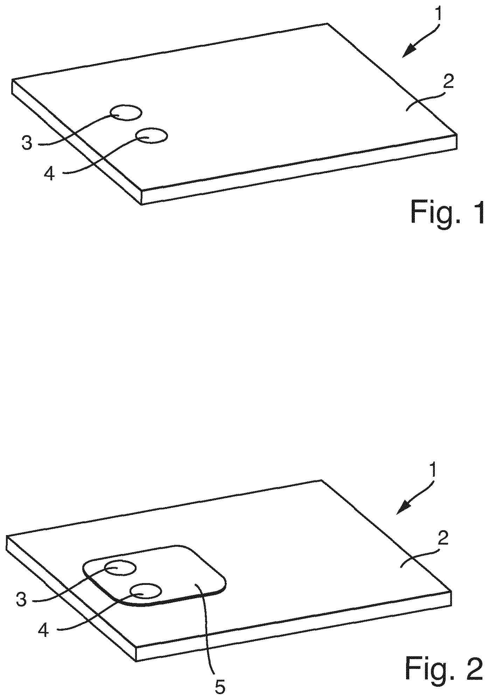

Die in Fig. 1-13 dargestellten Messanordnungen werden vorzugsweise in Messgeräten der Prozess- und Automatisierungstechnik eingesetzt.

Die vorliegende Erfindung betrifft die Anbindung von einem Sensor an ein Trägerelement. Diese Sensoren werden in den nachfolgenden Ausführungsbeispielen als mikromechanische Sensoren beschrieben. Die Erfindung ist allerdings nicht auf mikromechanische Sensoren beschränkt.

Die Grundfläche eines bevorzugten Sensors kann bevorzugt der maximalen Grundfläche eines Wafers entsprechen. Als Grundfläche ist dabei die Fläche zu verstehen, mit welcher der Sensor mit dem Trägerelement verbunden werden kann.

Besonders bevorzugt ist zumindest eine Kantenlänge des Sensors kleiner oder gleich 10cm. Ganz besonders bevorzugt sind alle Kantenlängen des Sensors kleiner oder gleich 10cm.

Fig. 1 zeigt ein zweites Ausführungsbeispiel einen Sensor 1, der als ein Sensor für ein Coriolis-Massendurchflussmessgerät welcher im vorliegenden Ausführungsbeispiel in mikromechanischer Bauweise (MEMS - Micro-Electro-Mechanical-System) ausgebildet ist. Der Sensor 1 umfasst einen Sensorkörper 2, der aus Keramik ausgebildet ist und eine Oberfläche mit einer ersten Anschlussöffnung 3 und einer zweiten Anschlussöffnung 4, die jeweils einen Durchmesser von ca. 1 mm umfassen, aufweist. Der Sensorkörpers 2 ist quaderförmig und weist eine erste und eine zweite quadratförmige Seitenfläche auf, die jeweils typischerweise ca. 1 cm² groß sind. Die erste und die zweite Anschlussöffnung 3, 4 sind an einer ersten Seitenfläche des Sensorkörpers 2 angeordnet und führen zu einem durchströmbaren Volumen, dass im Inneren des Sensorkörpers angeordnet ist und mittels eines Metallkörpers, insbesondere ein Metallrohr gegen den Sensorkörper abgegrenzt ist.

Fig. 2 zeigt einen ersten erfindungsgemäßen Verfahrensschritt. In diesem ersten Verfahrensschritt wird ein Teil der ersten Seitenfläche des Sensorkörpers 2 dermaßen galvanisiert, dass die galvanische Schicht 5 eine elektrische Verbindung sowohl zur ersten als auch zur zweiten Anschlussöffnung 3, 4 herstellt. Alternativ kann die gesamte Oberfläche des Sensorkörpers 2 galvanisiert werden. Auf diese Weise werden die elektrischen und elektronischen Komponenten des Sensors 1 gegen elektromagnetische Felder, die außerhalb des Sensorkörpers 2 herrschen, geschützt.

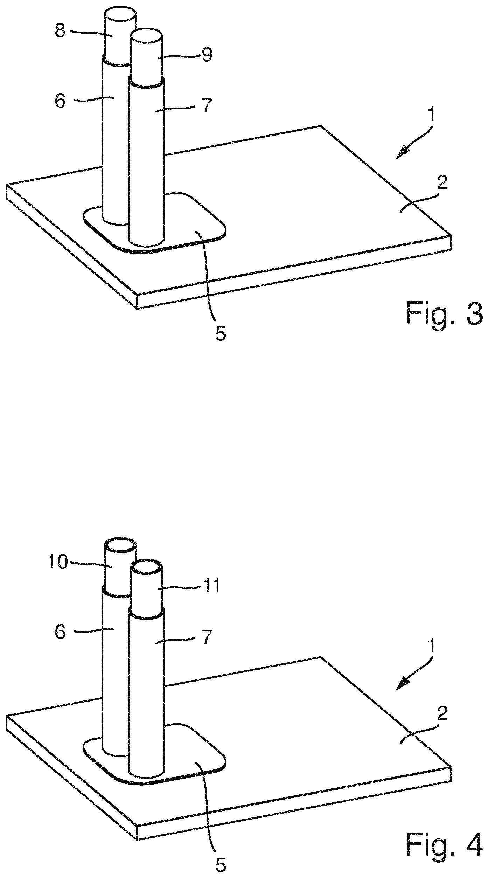

Fig. 3 zeigt den nächsten erfindungsgemäßen Verfahrensschritt. Ein erster und ein zweiter zylinderförmiger Leitungsrohr 6, 7, die jeweils eine Nennweite von typischerweise ca. 1 mm aufweisen und jeweils ein erster und ein zweiter Stöpsel 8, 9 aufweisen, werden jeweils senkrecht auf der ersten und der zweiten Anschlussöffnung 3, 4 positioniert. Der erste und der zweite Stöpsel 8, 9 sind zylinderförmig ausgebildet und weisen einen Außendurchmesser auf, der einem Innendurchmesser der Leitungsrohre 6, 7 entspricht und ragen jeweils zu einem Teil aus dem ersten und dem zweiten Leitungsrohr 6, 7 heraus, wobei das herausragende Teil des ersten und des zweiten Stöpsels 8, 9 an einem der ersten und der zweiten Anschlussöffnung 3, 4 gegenüberliegendes Ende der ersten und der zweiten Leitungsrohre 6, 7 liegt.

Der erste und der zweite Stöpsel 8, 9 dienen zum Verschließen des ersten und des zweiten Leitungsrohrs 6, 7 während des Galvanisierens keine Elektrolytische Flüssigkeit in den ersten und/oder zweiten Leitungsrohr 6, 7 gelangen kann. Alternativ kann auch der erste und der zweite Stöpsel 8, 9 direkt an die erste und zweite Anschlussöffnung 3, 4 angeordnet werden.

Fig. 4 zeigt, dass anstelle des ersten und des zweiten Stöpsels 8, 9 jeweils eine innere erste und zweite Röhre 10, 11 verwendet werden kann.

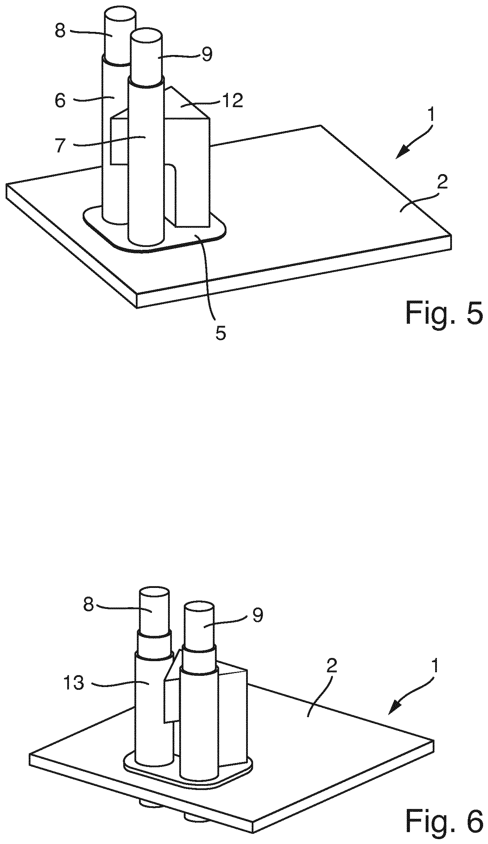

Fig. 5 zeigt, dass nach dem Positionieren des ersten und des zweiten Leitungsrohrs 6, 7, diese mittels einer Stütze 12 gegen die erste Seitenfläche des Sensorkörpers 2 gestützt werden. Die Stütze 12 kann z. B. mittels einer zweiten galvanischen Schicht auf der Seitenfläche des Sensorkörpers angeordnet werden.

Fig. 6 zeigt einen weiteren erfindungsgemäßen Verfahrensschritt, bei dem eine dichtende galvanische Beschichtung 13 auf der galvanischen Schicht 5 und einer Mantelfläche des ersten und des zweiten Leitungsrohrs 6, 7 erzeugt wird. Da die erste Seitenfläche des Sensorkörpers 2 aus Keramik besteht und nur zu einem Teil eine galvanische Schicht 5 aufweist, wird das Galvanisieren der ersten und der zweiten Anschlussöffnung 3, 4 nur den Teil der ersten Seitenfläche des Sensorkörpers 2 bedecken, die eine galvanische Schicht aufweist. Durch das Galvanisieren wird eine elektrische Verbindung zwischen dem ersten und dem zweiten Leitungsrohr 6, 7 und der ersten und der zweiten Anschlussöffnung 3, 4 hergestellt. Auf diese Weise können die durch Reibung des fließenden Mediums erzeugten elektrischen Felder, die im Inneren des Sensorkörpers 2 entstehen über den ersten und zweiten Leitungsrohr 6, 7 abfließen.

Wurden die Anschlussöffnungen 3, 4 mittels der Stopfen 8, 9 verschlossen, wird die galvanischen Beschichtung 13 auf der galvanischen Schicht 5 und einer Mantelfläche des ersten und des zweiten Stöpsels 8, 9 erzeugt. Nach dem Entfernen des ersten und zweiten Stöpsels 8, 9 bildet die Beschichtung 13, eine kommunizierende Verbindung zu der ersten und zweiten Anschlussöffnung und somit zu inneren Volumen des Sensorkörpers 2.

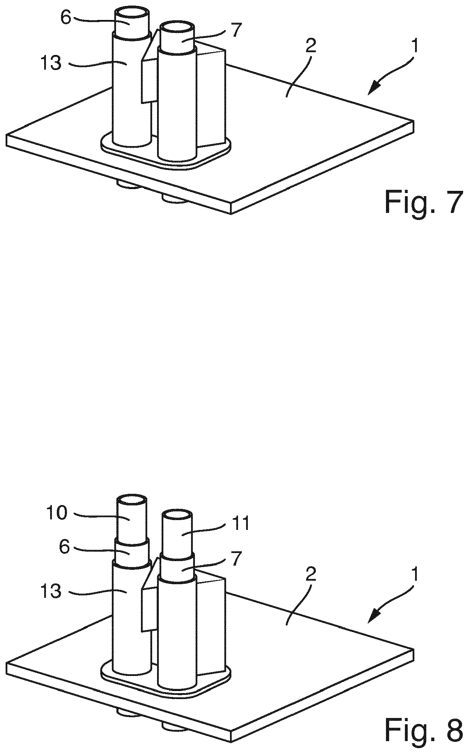

Fig. 7 zeigt einen weiteren erfindungsgemäßen Verfahrensschritt: Nach dem Erzeugen einer dichtenden galvanischen Beschichtung 13 zwischen einem Teil der Oberfläche des Sensorkörpers 2 und dem ersten und zweiten Leitungsrohr 6, 7 können der erste und der zweite Stöpsel 8, 9 entfernt werden. Dies kann mechanisch oder durch ein Auflösungs- und/oder Schmelzprozess geschehen.

Ist anstelle des ersten und des zweiten Stöpsels 8, 9 eine erste und eine zweite Röhre 10, 11 verwendet worden, so wird diese nicht entfernt (siehe Fig. 8). Die erste und zweite Röhre 10, 11 können dann an weiterführende Schläuche angeschlossen werden.

Durch das erfindungsgemäße Verfahren wird eine kommunizierende Verbindung zwischen dem Inneren durchströmbaren Volumen des Sensorkörpers 2 und dem ersten und zweiten Leitungsrohr 6, 7 geschaffen, so dass eine Druckänderung eines Mediums in dem ersten Leitungsrohr 6 über das Innere Volumen des Sensorkörpers 2 in das zweite Leitungsrohr 7 übertragen wird.

Anschließend kann der in Fig. 1-8 beschriebene Sensor mit einem Trägerelement zu einer erfindungsgemäßen Messanordnung verbunden werden.

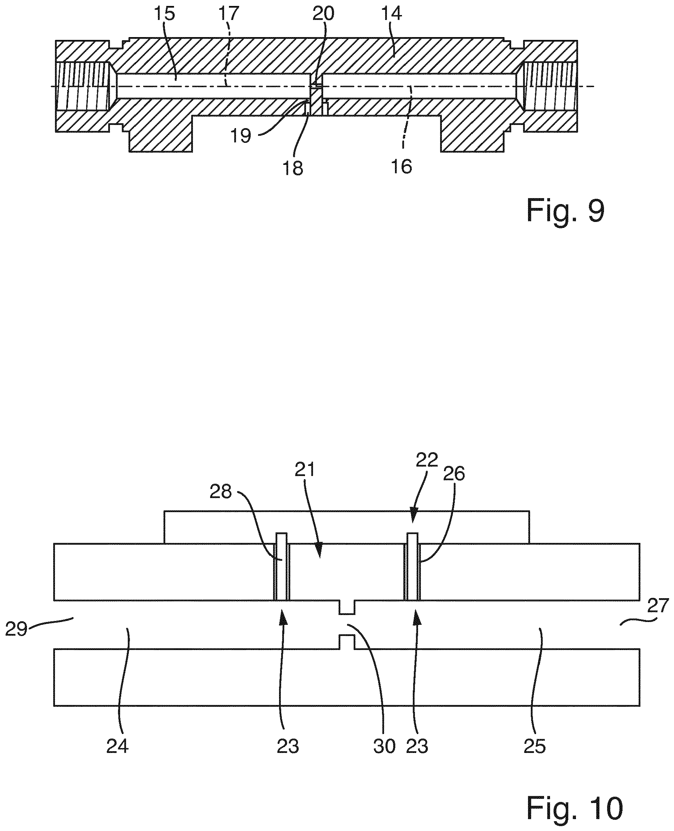

Die Leitungsrohre 6,7 können dabei auch Anschlusselemente sein, welche vor dem Festlegen im Sensorkörper an einem in Fig. 9 dargestelltes Trägerelement befestigt wurden. Die Festlegung der Leitungsrohre bzw. Anschlusselemente am Trägerelement soll nachfolgend näher erläutert werden.

Fig. 9 zeigt ein entsprechendes Trägerelement 14 mit einer Längsachse A auf welchem ein Sensor zur Ermittlung einer Prozessgröße eines gasförmigen oder flüssigen Fluids angeordnet werden kann. Das Trägerelement 14 weist einen Fluidkanal auf, welcher sich im vorliegenden Beispiel in einen Fluidzuführkanal 15 und einen Fluidabführkanal 16 zur Zu- und Abführung eines Fluids zum Sensor unterteilt. Es sind allerdings auch andere Trägerelement-Sensor Konstruktionen möglich, beispielsweise ein Drucksensor, bei welchem die Fluidzuführung und -abführung in einem Kanal zusammengefasst werden können.

Diese Prozessgröße kann vorzugsweise die Dichte, die Viskosität, die Stoffzusammensetzung, die Temperatur, pH-Wert, die Leitfähigkeit, der Partikelgehalt, der Volumendurchfluss, der Massendurchfluss und/oder die Durchflussgeschwindigkeit eines Fluids sein.

Der Fluidzuführkanal weist in dem in Fig. 9 dargestellten Ausführungsbeispiel einen erstes Kanalsegment 17 auf, welches im Wesentlichen parallel zur Längsachse A des Trägerelements 14 verläuft. Dieses Kanalsegment ist endständig mit einem Prozessanschluss einer Rohrleitung verbindbar. Der Fluidzuführkanal weist zudem ein zweites Kanalsegment 18 in welches das erste Kanalsegment 17 mündet. Dieses zweite Kanalsegment 18 ist im vorliegenden Ausführungsbeispiel im Winkel von 90° zur Längsachse im Trägerelement 14 angeordnet. Dabei ist der Durchmesser des ersten Kanalsegments 17 größer, vorzugsweise zumindest doppelt so groß, wie der Durchmesser des zweiten Kanalsegments 18. Das zweite Kanalsegment 18 weist eine Durchmesseraufweitung 19 zur Aufnahme eines Abschlusselements auf. Dadurch erfolgt nach dem Einsetzen des Anschlusselements kein Nennweitensprung innerhalb des zweiten Kanalsegments 18. Durch das zweite Kanalsegment 18 kann das Fluid radial zur Achse aus dem Trägerelement herausgeleitet werden.

Das Trägerelement 14 weist zudem den Fluidabführkanal 16 als Teil des Fluidkanals auf, welcher im Wesentlichen baugleich zum Fluidzuführkanal 15 aufgebaut ist. Zwischen dem Fluidabführkanal und dem Fluidzuführkanal kann optional ein Kanalverbindungssegment 20 angeordnet sein, welches im Trägerelement 14 parallel zur Längsachse A angeordnet ist und den Fluidzuführkanal und den Fluidabführkanal miteinander verbindet. Somit muss nicht der gesamte Fluidstrom durch den Sensor geleitet werden, sondern nur ein Teil des Fluids. Die Nennweite des Kanalverbindungssegments weist dabei einen kleineren Durchmesser, vorzugsweise zumindest einen doppelt so kleinen Durchmesser auf wie das erste Kanalsegment 17.

Fig. 10 gibt einen schematischen Aufbau einer ersten Messanordnung 31 wieder. Eine erfindungsgemäße Messanordnung umfasst zumindest ein Trägerelement 21, einen mikroelektromechanischen Sensor 22 und Anschlusselemente 23, welche das Trägerelement 21 und den Sensor 22 miteinander verbindet, derart, dass das Anschlusselement 23 in den mikroelektromechanischen Sensor 22, respektive in einen darin angeordneten Fluidkanal, hineinragt.

Mikroelektromechanische Sensoren, wie sie im vorliegenden Beispiel eingesetzt werden können, sind an sich bekannt. Die im vorliegenden Beispiel eingesetzten Sensoren können als Coriolis-Durchflussmessgerät, als magnetisch-induktives Durchflussmessgerät, als thermisches Durchflussmessgerät, als Druckmessgerät, als Viskositätsmessgerät spektroskopische Messgeräte, Ultraschallmessgeräte, insbesondere Ultraschall-Durchflussmessgerät, Dichtemessgeräteausgebildet sein und Prozessgrößen wie Viskosität, Dichte, Druck, Stoffzusammensetzung, Temperatur, Viskosität, der pH-Wert, die Leitfähigkeit, der Partikelgehalt Und/oder ggf. auch Durchfluss ermittelt. Unter Sensoren sind im Rahmen der vorliegenden Erfindung auch chromatographische Abalysatoren (LC- oder GC-Analysatoren) zu verstehen. Diese sind ebenfalls in mikroelektromechanischer Bauweise realisierbar.

Das Trägerelement 21 weist einen Fluidzuführkanal 24 und einen Fluidabführkanal 25 auf. Diese weisen jeweils ein erstes Kanalsegment 27, 29 parallel zur Längsachse A auf und ein zweites Kanalsegment 26 und 28, welches sich radial zur Längsachse durch das Trägerelement 21 erstreckt. Auch in dem in Fig. 10 aufgeführten Ausführungsbeispiel ist zwischen den jeweils ersten Kanalsegmenten 27 und 29 des Fluidzuführkanals 24 und des Fluidabführkanals 25 ein Kanalverbindungssegment 30 angeordnet.

Im Unterschied zu Fig. 9 weist das Trägerelement 21 keine Durchmesseraufweitung 19 auf. Vielmehr sind die Anschlusselemente 23 vollständig in die zweiten Kanalsegmente 26 oder 28 eingesetzt.

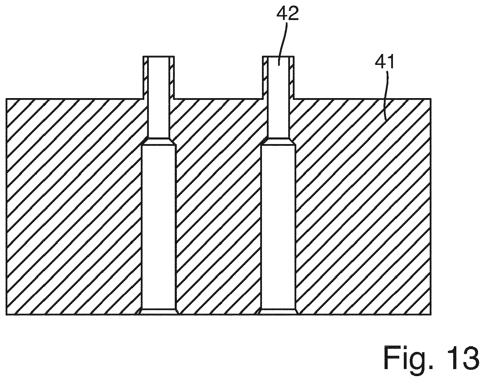

Der mikroelektromechanische Sensor ist vorzugsweise aus einem Glas oder Siliziummaterial gefertigt. Typischerweise beträgt der Temperaturausdehnungskoeffizient bei diesen Materialien etwa 3*10⁻⁶ K⁻¹. Alternativ sind auch Sensoren aus keramischen Materialien für diesen Einsatzzweck verwendbar. Die Anschlusselemente 23 sind entweder als gesonderte Bauteile in Form von Röhrchen ausgebildet oder integral ausgeformt, wie dies in Fig. 13 näher erläutert wird. Sie bestehen vorzugsweise aus Edelstahl - vorzugsweise der Sorte PH 17-4. Andere Materialien, beispielsweise aus Kunststoffmaterialien, sind allerdings ebenfalls denkbar. Gerade bei besonders heißen oder kalten Fluiden ist es jedoch von Vorteil, wenn der thermische Ausdehnungskoeffizient des Materials des Sensors und des Anschlusselements um nicht mehr als das 5-fache voneinander abweichen. Andernfalls kann es zu Undichtigkeiten bei höheren Drücken oder sogar zu einem Ablösen des Sensors kommen. Edelstahl der Sorte PH 17-4 erfüllt diese Anforderungen bezüglich eines Siliziummaterials und/oder Glasmaterials (incl. Borsilikat). Sofern die Anschlusselemente integral mit dem Trägerelement ausgebildet sind sollte das Material des Trägerelements naturgemäß dem Material der Anschlusselemente entsprechen. Sofern jedoch die Anschlusselemente 23 als gesonderte Bauteile im Trägerelement 21 vorgesehen sind, so kann das Material des Trägerelements vorzugsweise aus einem kostengünstigeren Material, beispielsweise Edelstahl der Sorte 316 L ausgewählt werden. Alternativ kann auch anderes Material, insbesondere Titan, Aluminium, Zirkon, Tantal, Silizium oder leitendes Keramikmaterial für das Trägerelement und/oder das Anschlusselement eingesetzt werden.

Zusätzlich zur metallischen Anbindungsschicht kann auch eine Kunststoffschicht vorgesehen sein, welche die Anbindungsschicht vor oxidativem Angriff schützt. Dabei kann es sich bevorzugt um ein Copolymer handeln.
In einer besonderen Ausführungsvariante besteht eine Innenbeschichtung des Anschlusselements oder das Anschlusselement insgesamt aus einem Kunststoff ausgewählt aus folgenden Stoffen: PE, PEEK, PFA, PTFE, PBT PEK. Hier muss allerdings im Falle der Herstellung einer galvanischen Beschichtung zunächst eine elektrisch leitfähige Schicht in Form von Sputtern, Metallisieren oder Aufdampfen aufgebracht werden.

Zusätzlich oder alternativ können auch wärmeleitfähigen Materialien, welche die Wärmeleitfähigkeit der metallischen Anbindungsschicht erhöhen, in den diese Anbindungsschicht eingebunden werden um eine thermische Kontaktierung zwischen dem Trägerelement und dem Sensor zu ermöglichen.

Zusätzlich oder alternativ können auch magnetische Substanzen in den Kunststoff eingebunden werden, um die magnetische Kontaktierung zwischen Sensor und Trägerelement zu ermöglichen. Entsprechende magnetische Substanzen kann z.B. Partikel aus Magneteisenstein sein.

Auch metallische Elemente, beispielsweise Leiterbahnen, welche die elektrische Leitfähigkeit verbessern können in der metallische Anbindungsschicht enthalten sein
Zwischen dem Trägerelement und dem Anschlusselement und dem Trägerelement und dem Sensor kann zudem vorteilhaft eine Vordichtung in Form einer Membranstruktur oder einer Dichtlippe angeordnet sein, so dass die Lotverbindung mechanisch oder chemisch nicht übermäßig beansprucht wird.

Sofern eine vorgenannte Lotverbindung geschaffen wird, empfiehlt es sich zuvor die zu verbindenden Oberflächen zu behandeln, um ein besseres Anhaften zu ermöglichen. Dies kann chemisch durch Anätzen erfolgen oder durch Coronabestrahlen oder Lasern oder durch abrasive Verfahren wie z.B. Sandstrahlen. Die behandelten Oberflächen können sodann durch das Lot besser benetzt werden.

Die Anschlusselemente 23 ermöglichen insbesondere einen strömungstechnischen Anschluss zwischen mikroelektromechanischem Sensor 22 und dem Trägerelement 21. Allerdings empfiehlt sich, insbesondere bei höheren Drücken, eine zusätzliche mechanische Anbindung des mikroelektromechanischen Sensors 22.

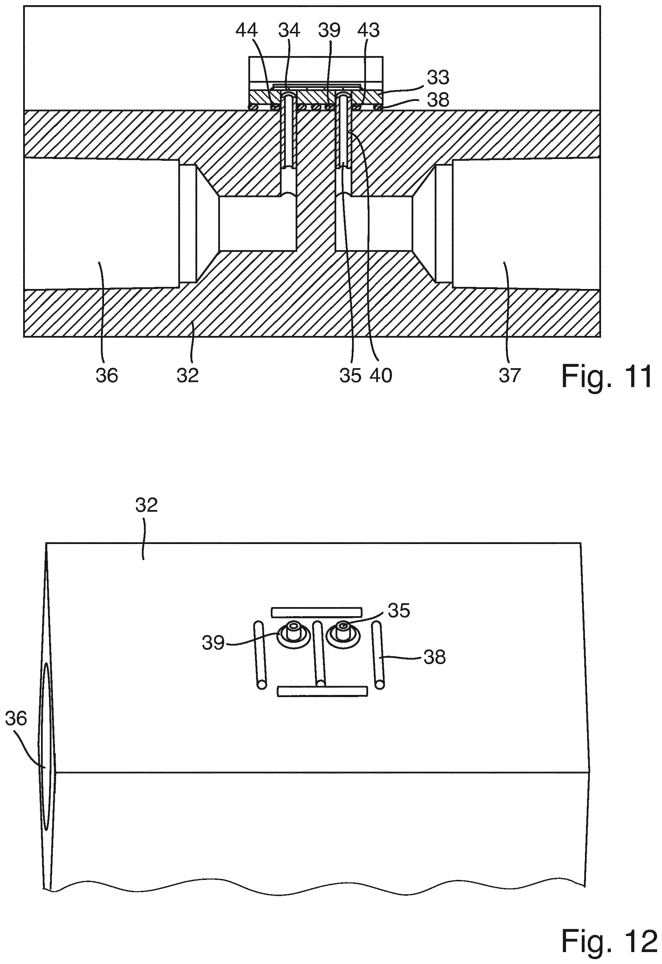

Eine Variante der mechanischen Anbindung wird in den Fig. 11 und 12 näher erläutert. Fig. 11 und 12 zeigen in Analogie zur Fig. 10 ein Trägerelement 32 mit einem Fluidzuführkanal 36 und einem Fluidabführkanal 37. In diesen Kanälen 36 und 37 sind jeweils Anschlusselemente 35 angeordnet. Fig. 11 zeigt zudem einen mikroelektromechanischen Sensor 33 mit einem darin angeordneten Fluidkanal 34, welcher sich von einem Fluideinlass 44 bis zu einem Fluidauslass 43 erstreckt und in welchen die Anschlusselemente 35 hineinragen.

Die mechanische Anbindung des mikroelektromechanischen Sensors erfolgt im Ausführungsbeispiel der Fig. 11 und 12 mittels einer Lotverbindung. Diese Lotverbindung ist in Fig. 11 und 12 in Form von Lotdrähten 38 und Lotringen 39 auf das Trägerelement 32 aufgebracht. Durch die Lotringe 39 wird eine mechanische und zugleich druckstabile und mediumsdichte Verbindung der Anschlusselemente 35 mit dem Trägerelement 32 erreicht.

Die Anbindung zwischen den mikroelektromechanischen Sensor und dem Trägerelement kann alternativ zu einer Lotverbindung auch durch ein Klebsystem, z.B. mittels eines Epoxyharzes erfolgen. Die Lotverbindung ist allerdings besonders stabil gegenüber Säuren und Laugen.

Zusätzlich zu den Lotringen 39 sind auch Lotdrähte auf dem Trägerelement 32 aufgebracht, welche eine direkte Verbindung mit dem mikroelektromechanischen Sensor 33 ermöglichen.

Als Lotmaterial eignet sich besonders bevorzugt ein Edelmetall, z.B. Silber oder Gold oder Legierungen daraus. Es ist beispielsweise auch möglich eutektische Gemische aus Silber und Zinn einzusetzen. Die Schrumpfung dieser Materialien beträgt dabei vorzugsweise weniger als 1 Vol.%

Alternativ oder zusätzlich zu den Lötringen und Lötdrähten können auch gestanzte Metallfolien, insbesondere Gold und/oder Zinnfolien, und/oder eine elektrochemisch-abgeschiedene Schicht oder Schichten, insbesondere eine Goldschicht, für eine sichere Anbindung sorgen. Das Lot kann zudem mittels einer Schablone auf das Trägermaterial aufgebracht werden.

Bei der elektrochemischen Abscheidung kann zur gezielteren Auftragung der Schicht ein Teil der Oberfläche des Trägerelements 32 maskiert werden. Dies garantiert eine definierte Höhe des Lotes und ein definiertes Volumen des Lotes.

Alternativ jedoch weniger bevorzugt zu Goldmaterial kann auch Zinnmaterial oder Legierungen aus beiden Materialien für die Ausbildung der Lotverbindungen genutzt werden. Sowohl Gold als auch Zinn weisen eine gute chemische Beständigkeit gegenüber den meisten Fluiden auf. Die Schrumpfung dieser Materialien beträgt dabei vorzugsweise weniger als 1 Vol.%.

Dabei ist es von Vorteil, wenn die Lotschicht geringer als 1/5 mm, vorzugsweise geringer als 1/10 mm ist.

Eine elektrochemische Abscheidung einer metallischen Schicht, kann analog zu dem in Fig. 1-8 beschriebenen Ausführungsbeispiel mittels einer galvanischen Abscheidung erfolgen.

Alternativ kann eine mehrschichtige elektrochemische Abscheidung erfolgen, wobei die Goldschicht oder Zinnschicht lediglich die zum Sensor hin oberste Schicht ist.

Im Falle einer galvanischen Abscheidung einer metallischen Anbindungsschicht auf dem Sensor, dem Trägerelement und/oder den Anschlusselementen kann zur Verbesserung der Abscheidungsrate und der Anhaftung ein Leitlack, vorzugsweise ein Silber- oder Graphitleitlack, eingesetzt werden.

Analog zur Verbindung zwischen dem Trägerelement 32 und einem der Anschlusselemente 35 kann auch eine Verbindung zwischen dem mikroelektromechanischen Sensor und einem der Anschlusselemente 39 erreicht werden.

Besonders wegen ihrer mechanischen Stabilität ist dabei eine einheitliche metallische Anbindungsschicht, welche sich vom Trägerelement 32 über das Anschlusselement 35 bis zum mikroelektromechanischen Sensor 33 erstreckt.

Die in Fig. 1-13 dargestellte fluidleitende Verbindung, welche durch das Anschlusselement bereitgestellt wird, weist vorzugsweise eine Querschnittsfläche von weniger als 100 mm² und insbesondere weniger als 20mm² auf.

Eine bevorzugte Schichtdicke der Anbindungsschicht beträgt weniger als 1 mm, vorzugsweise weniger als 200 µm und besonders bevorzugt weniger als 100µm. Eine besonders bevorzugte Schichtdicke der mechanischen Anbindungsschicht liegt im Bereich zwischen 100nm und 100µm,

Fig. 13 zeigt Anschlusselemente 42 zur Anbindung des Sensorelements an das Trägerelement 41, welche allerdings integral mit dem Trägerelement 41 verbunden sind.

Dieser integrale Aufbau ist insbesondere von Vorteil, da bereits eine Undichtigkeitsstelle bei dieser Ausführungsvariante vermieden wird. Eine mögliche Herstellung eines solchen integral ausgebildeten Anschlusselements kann beispielsweise mittels eines Kaltformverfahrens, insbesondere mittels Fräsen, ausgeformt werden.

Durch die Anordnung von Anschlusselementen zwischen dem Trägerelement und dem mikroelektromechanischen Sensor und dem zusätzlichen Einstecken in den mikroelektromechanischen Sensor, wird gegenüber einem herkömmlichen Lötstopp ein definierteres Totvolumen geschaffen. Zudem wird eine reproduzierbarere und chemisch beständigere Verbindung erreicht, welche zudem stabiler gegenüber Scherkräften ist.

Dabei wirkt das Anschlusselement bei Auftrag eines Lotes wie ein definierter Lotstop ohne dass sich ein Totvolumen ausbildet.

Die derart geschaffene Anbindung eines Sensors, welcher beispielsweise in mikroelektromechanischer Bauweise ausgeführt ist, an das Trägerelement ist vorzugsweise druckstabil bis zu einem Druck von mehr als 20 bar, vorzugsweise mehr als 80 bar.

Verbindungen, welche die elektrische, thermische und/oder magnetische Leitfähigkeit der Anbindungsschicht verbessern können der metallischen Anbindungsschicht zugesetzt werden. Alternativ oder zusätzlich können auch Verbindungen, welche eine bessere Wärmeausdehnungsanpassung zwischen den Materialien des Trägerelements und des Sensors ermöglichen dem Metall der Anbindungsschicht zugesetzt werden.

Verbindung zur Verbesserung der elektrischen Leitfähigkeit sind bevorzugt lötfähige und zugleich leitfähige Verbindungen, wie die bereits zuvor genannten Verbindungen,
Verbindungen welche die thermische Leitfähgikeit verbessern können beispielsweise Siliziumcarbid und/oder Aluminiumnitrid sein.

Verbindungen, welche eine bessere Wärneausdehnungsanpassung ermöglichen können vorzugsweise Korund und/oder Aluminiumoxid sein.

Verbindungen welche die magnetische Leitfähigkeit verbessern können beispielsweise Magneteisenstein oder magnetisierbare Metalle oder Metalllegierungen sein.

### Bezugszeichenliste

- 1: Sensor
- 2: Sensorkörper
- 3: Erste Anschlussöffnung
- 4: Zweite Anschlussöffnung
- 5: Erste Galvanische Schicht
- 6: Erstes Leitungsrohr
- 7: Zweites Leitungsrohr
- 8: Erster Stöpsel
- 9: Zweiter Stöpsel
- 10: Erste Röhre
- 11: Zweite Röhre
- 12: Stütze
- 13: Galvanische Beschichtung
- 14: Trägerelement
- 15: Fluidzuführkanal
- 16: Fluidabführkanal
- 17: erstes Kanalsegment
- 18: zweites Kanalsegment
- 19: Durchmesseraufweitung
- 20: Kanalverbindungssegment
- 21: Trägerelement
- 22: Mikroelektromechanischer Sensor
- 23: Anschlusselement
- 24: Fluidzuführkanal
- 25: Fluidabführkanal
- 26: Zweites Kanalsegment
- 27: Erstes Kanalsegment
- 28: Zweites Kanalsegment
- 29: Erstes kanalsegment
- 30: Kanalverbindungssegment
- 31: Messanordnung
- 32: Trägerelement
- 33: Mikroelektromechanischer Sensor
- 34: Fluidkanal
- 35: Anschlusselement
- 36: Fluidzuführkanal
- 37: Fluidabführkanal
- 38: Lotdrähte
- 39: Lotringe
- 40: Fluidauslass
- 41: Trägerelement
- 42: Anschlusselement
- 43: Fluidauslass
- 44: Fluideinlass

## Patentansprüche

1. Messanordnung umfassend
a) ein Trägerelement (14, 21, 32, 41) mit einer Längsachse (A) auf welchem ein Sensor (1, 22, 33), zur Ermittlung einer Prozessgröße eines gasförmigen oder flüssigen Fluids angeordnet ist, und
b) den Sensor (1, 22, 33) wobei der Sensor (1, 22, 33) einen Fluidkanal (34) aufweist, welcher sich innerhalb des Sensors (1, 22, 33) erstreckt, und wobei das Trägerelement (14, 21, 32, 41) einen Fluidkanal aufweist, wobei das Trägerelement (14, 21, 32, 41) zur Verbindung des Fluidkanals des Trägerelements (14, 21, 32, 41) mit dem Fluidkanal (34) des Sensors (1, 22, 33) jeweils zumindest ein Anschlusselement (6. 7, 23, 35, 42) aufweist, welches senkrecht zur Längsachse (A) aus dem Trägerelement (14, 21, 32, 41) hervorsteht und welches in den Fluidkanal (34) des Sensors (1, 22, 33) hineinragt, **dadurch gekennzeichnet, dass** das Anschlusselement (42) einstückig mit dem Trägerelement (41) verbunden ist und der Sensor durch ein Klebsystem oder eine Lotverbindung mit dem Trägerelement verbunden ist.

2. Messanordnung nach Anspruch 1, **dadurch gekennzeichnet, dass** der Sensor durch das Klebsystem mittels eines Epoxyharzes mit dem Trägerelement verbunden ist.

3. Messanordnung nach Anspruch 1, **dadurch gekennzeichnet, dass** das Anschlusselement (42) mittels eines Urformverfahrens aus dem Trägerelement (41) ausgeformt ist.

4. Messanordnung nach Anspruch 1, **dadurch gekennzeichnet, dass** das Anschlusselement (6, 7, 23, 35) als ein rohrförmiges Bauteil ausgebildet ist, welches in dem Fluidkanal des Trägerelements (14, 21, 32) angeordnet ist.

5. Messanordnung nach Anspruch 4, **dadurch gekennzeichnet, dass** das Anschlusselement (6, 7, 23, 35) an einer Austrittsöffnung des Fluidkanals eine metallische Anbindungsschicht zur mechanischen Anbindung des Anschlusselements (6, 7, 23, 35) an das Trägerelement (14, 21, 32) aufweist, welche sich über einen Teilbereich einer Oberfläche des Trägerelements (14, 21, 32) erstreckt.

6. Messanordnung nach Anspruch 2, 3 oder 5, **dadurch gekennzeichnet, dass** sich eine metallische Anbindungsschicht oder die metallische Anbindungsschicht gemäß Anspruch 5 über einen Teilbereich einer Oberfläche des Sensors (1, 22, 33) erstreckt, zur mechanischen Anbindung des Anschlusselements (6, 7, 23, 35) an den Sensor (1, 22, 33).

7. Messanordnung nach Anspruch 5 oder 6, **dadurch gekennzeichnet, dass** die metallische Anbindungsschicht als eine Lotverbindung ausgebildet ist.

8. Messanordnung nach Anspruch 7, **dadurch gekennzeichnet, dass** die Lotverbindung durch Schmelzen eines Lotdrahtes oder besonders bevorzugt einer vorgestanzten Lotfolie oder einer elektrochemisch abgeschiedenen Lotbeschichtung hergestellt ist.

9. Messanordnung nach Anspruch 8, **dadurch gekennzeichnet, dass** zumindest eine Materialkomponente der Lotverbindung ein Edelmetall, insbesondere Gold, und/oder Zinn ist.

10. Messanordnung nach Anspruch 5, **dadurch gekennzeichnet, dass** die metallische Anbindungsschicht in Form einer elektrochemisch-abgeschiedenen Metallschicht ausgebildet ist.

11. Messanordnung nach Anspruch 8, **dadurch gekennzeichnet, dass** die elektrochemisch-abgeschiedene Metallschicht als galvanische Beschichtung (13) ausgebildet ist und dass zwischen dem Trägerelement (14, 21, 32, 41) und der galvanischen Beschichtung eine Schicht aus einem Leitlack angeordnet ist.

12. Messanordnung nach einem der vorhergehenden Ansprüche, **dadurch gekennzeichnet, dass** das Trägerelement (14, 21, 32, 41) und/oder das Anschlusselement (6, 7, 23, 35, 42) aus Metall, vorzugsweise aus Edelstahl, besonders bevorzugt aus Edelstahl der Sorte PH 17-4 besteht.

13. Messanordnung nach einem der vorhergehenden Ansprüche, **dadurch gekennzeichnet, dass** der thermische Ausdehnungskoeffizient des Materials des Trägerelements (14, 21, 32, 41) weniger als das 5-fache, vorzugsweise weniger als das 4-fache des thermischen Ausdehnungskoeffizienten des Materials des Sensors (1, 22, 33) beträgt.

14. Messanordnung nach einem der vorhergehenden Ansprüche, **dadurch gekennzeichnet, dass** zwischen dem Sensor (1, 22, 33) und dem Trägerelement (14, 21, 32, 41) weitere stoffschlüssige Verbindungen angeordnet sind, wobei die stoffschlüssigen Verbindungen insbesondere als Lotverbindungen (39) ausgebildet sind.

15. Messanordnung nach Anspruch 13 oder 14, **dadurch gekennzeichnet, dass** die dem Trägerelement (14, 21, 32, 41) zugewandte Oberfläche des Sensors (1, 22, 33) in zumindest drei gleich-dimensionierte Sensorabschnitte einteilbar sind, wobei zumindest zwei der drei Sensorabschnitte zumindest eine jeweils eine der stoffschlüssigen Verbindungen aufweisen.

## Claims

1. Measuring arrangement comprising
a) a support element (14, 21, 32, 41) with a longitudinal axis (A) on which a sensor (1, 22, 33) is arranged to determine a process variable of a gaseous or liquid fluid, and
b) the sensor (1, 22, 33)
wherein the sensor (1, 22, 33) has a fluid channel (34), which extends inside the sensor (1, 22, 33), and
wherein the support element (14, 21, 32, 41) has at least one connection element (6, 7, 23, 35, 42) for connecting the fluid channel of the support element (14, 21, 32, 41) with the fluid channel (34) of the sensor (1, 22, 33), said connection element projecting out of the support element (14, 21, 32, 41) in a manner perpendicular to the longitudinal axis (A) and projecting into the fluid channel (34) of the sensor (1, 22, 33), **characterized in that** the connection element (42) is connected in one piece to the support element (41) and **in that** the sensor is connected to the support element by an adhesive system or a solder connection.

2. Measuring arrangement as claimed in Claim 1, **characterized in that** the sensor is connected to the support element by the adhesive system by means of an epoxy resin.

3. Measuring arrangement as claimed in Claim 1, **characterized in that** the connection element (42) is formed from the support element (41) by means of an original molding process.

4. Measuring arrangement as claimed in Claim 1, **characterized in that** the connection element (6, 7, 23, 35) is designed as a tubular component that is arranged in the fluid channel of the support element (14, 21, 32).

5. Measuring arrangement as claimed in Claim 4, **characterized in that** the connection element (6, 7, 23, 35) has a metal connection layer on an outlet opening of the fluid channel for the mechanical connection of the connection element (6, 7, 23, 35) to the support element (14, 21, 32), said layer extending over a part of a surface of the support element (14, 21, 32).

6. Measuring arrangement as claimed in Claim 2, 3 or 5, **characterized in that** a metal connection layer or the metal connection layer as claimed in Claim 5 extends over a part of a surface of the sensor (1, 22, 33) to mechanically connect the connection element (6, 7, 23, 35) to the sensor (1, 22, 33).

7. Measuring arrangement as claimed in Claim 5 or 6, **characterized in that** the metal connection layer is designed as a solder connection.

8. Measuring arrangement as claimed in Claim 7, **characterized in that** the solder connection is established by the fusion of a solder wire, or particularly preferably, by the fusion of a pre-cut solder foil or an electrochemically deposited solder layer.

9. Measuring arrangement as claimed in Claim 8, **characterized in that** at least one material component of the solder connection is a noble metal, particularly gold, and/or tin.

10. Measuring arrangement as claimed in Claim 5, **characterized in that** the metal connection layer is designed in the form of an electrochemically deposited metal layer.

11. Measuring arrangement as claimed in Claim 8, **characterized in that** the electrochemically deposited metal layer is designed as a galvanic coating (13) and **in that** a coat of a conductive varnish is arranged between the support element (14, 21, 32, 41) and the galvanic coating.

12. Measuring arrangement as claimed in one of the previous claims, **characterized in that** the support element (14, 21, 32, 41) and/or the connection element (6, 7, 23, 35, 42) is made from metal, particularly from stainless steel, particularly preferably from stainless steel of the type PH 17-4.

13. Measuring arrangement as claimed in one of the previous claims, **characterized in that** the thermal expansion coefficient of the material of the support element (14, 21, 32, 41) is less than 5 times, preferably less than 4 times, the thermal expansion coefficient of the material of the sensor (1, 22, 33).

14. Measuring arrangement as claimed in one of the previous claims, **characterized in that** additional bonded connections are arranged between the sensor (1, 22, 33) and the support element (14, 21, 32, 41), wherein the bonded connections are particularly designed as solder connections (39).

15. Measuring arrangement as claimed in Claim 13 or 14, **characterized in that** the surface of the sensor (1, 22, 33) facing towards the support element (14, 21, 32, 41) can be divided into at least three sensor sections of the same size, wherein at least two of the three sensor sections have at least one of the bonded connections.

## Revendications

1. Dispositif de mesure comprenant
a) un élément support (14, 21, 32, 41) avec un axe longitudinal (A) sur lequel est disposé un capteur (1, 22, 33), destiné à la détermination d'une grandeur process d'un fluide gazeux ou liquide, et
b) le capteur (1, 22, 33)
pour lequel le capteur (1, 22, 33) comporte un passage de fluide (34), qui s'étend à l'intérieur du capteur (1, 22, 33), et
pour lequel l'élément support (14, 21, 32, 41) comporte, pour la liaison du passage de fluide de l'élément support (14, 21, 32, 41) avec le passage de fluide (34) du capteur (1, 22, 33), au moins un élément de raccordement (6, 7, 23, 35, 42), qui déborde de l'élément support (14, 21, 32, 41) perpendiculairement à l'axe longitudinal (A) et qui pénètre dans le passage de fluide (34) du capteur (1, 22, 33), **caractérisé en ce que** l'élément de raccordement (42) est relié en une pièce avec l'élément support (41) et **en ce que** le capteur est relié avec l'élément support par un système de collage ou une liaison soudée.

2. Dispositif de mesure selon la revendication 1, **caractérisé en ce que** le capteur est relié avec l'élément support par le système de collage au moyen d'une résine époxy.

3. Dispositif de mesure selon la revendication 1, **caractérisé en ce que** l'élément de raccordement (42) est formé à partir de l'élément support (41) au moyen d'un procédé de formage.

4. Dispositif de mesure selon la revendication 1, **caractérisé en ce que** l'élément de raccordement (6, 7, 23, 35) est conçu en tant que composant tubulaire, lequel est disposé dans le passage de fluide de l'élément support (14, 21, 32).

5. Dispositif de mesure selon la revendication 4, **caractérisé en ce que** l'élément de raccordement (6, 7, 23, 35) présente sur une ouverture de sortie du passage de fluide une couche de liaison métallique pour la liaison de l'élément de raccordement (6, 7, 23, 35) à l'élément support (14, 21, 32), laquelle couche s'étend sur une zone partielle d'une surface de l'élément support (14, 21, 32).

6. Dispositif de mesure selon la revendication 2, 3 ou 5, **caractérisé en ce que** s'étend une couche de liaison métallique ou la couche de liaison métallique selon la revendication 5 sur une zone partielle d'une surface du capteur (1, 22, 33), pour la liaison mécanique de l'élément de raccordement (6, 7, 23, 35) sur le capteur (1, 22, 33).

7. Dispositif de mesure selon la revendication 5 ou 6, **caractérisé en ce que** la couche d'application métallique est conçue en tant que liaison soudée.

8. Dispositif de mesure selon la revendication 7, **caractérisé en ce que** la liaison soudée est obtenue par la fusion d'un fil à braser ou, de préférence, par la fusion d'une feuille à braser ou d'une couche à braser électrodéposée.

9. Dispositif de mesure selon la revendication 8, **caractérisé en ce qu'**au moins un composant du matériau de la liaison soudée est un métal précieux, notamment de l'or et/ou de l'étain.

10. Dispositif de mesure selon la revendication 5, **caractérisé en ce que** la couche de liaison métallique est conçue sous la forme d'une couche métallique électrodéposée.

11. Dispositif de mesure selon la revendication 8, **caractérisé en ce que** la couche métallique électrodéposée est conçue en tant que revêtement galvanique (13) et **en ce qu'**une couche de vernis conducteur est disposée sur le revêtement galvanique.

12. Dispositif de mesure selon l'une des revendications précédentes, **caractérisé en ce que** l'élément support (14, 21, 32, 41) et/ou l'élément de raccordement (6, 7, 23, 35, 42) est en métal, de préférence en acier inoxydable, particulièrement de préférence en acier inoxydable de la sorte PH 17-4.

13. Dispositif de mesure selon l'une des revendications précédentes, **caractérisé en ce que** le coefficient de dilatation thermique du matériau de l'élément support (14, 21, 32, 41) est inférieur à 5 fois, de préférence inférieur à 4 fois le coefficient de dilatation thermique du matériau du capteur (1, 22, 33).

14. Dispositif de mesure selon l'une des revendications précédentes, **caractérisé en ce qu'**est disposé, entre le capteur (1, 22, 33) et l'élément support (14, 21, 32, 41), d'autres liaisons par adhésion de matière, les liaisons par adhésion de matière étant notamment conçues en tant que liaisons soudées (39).

15. Dispositif de mesure selon la revendication 13 ou 14, **caractérisé en ce que** la surface de capteur (1, 22, 33) faisant face à l'élément support (14, 21, 32, 41) est divisible en au moins trois parties de capteur de dimensions égales, au moins deux des trois parties de capteur présentant au moins l'une des liaisons par adhésion de matière.
